# EUROPEAN PATENT SPECIFICATION

(11) **EP 1 464 356 B1**
(45) Date of publication and mention of the grant of the patent: **22.08.2007**
(21) Application number: 04016150.7
(22) Date of filing: 23.07.2001
(51) Int. Cl.: A61M 15/00

(54) **Inhalator**
Inhalator
Inhalateur

(30) Priority: 27.11.2000 JP 2000359822; 29.11.2000 JP 2000363636
(43) Date of publication of application: 06.10.2004
(62) Divisional of application: 01117861.3
(73) Proprietor: Hitachi, Ltd., Tokyo 100-8280 (JP)
(72) Inventor: Ohki, Hisatomo, Atsugi-shi Kanagawa 243-8510 (JP); Yazawa, Yoshiyuki, Atsugi-shi Kanagawa 243-8510 (JP); Nakamura, Shigemi, Atsugi-shi Kanagawa 243-8510 (JP); Yokobori, Mitsuru, Atsugi-shi Kanagawa 243-8510 (JP); Ishizeki, Kazunori, Atsugi-shi Kanagawa 243-8510 (JP)
(74) Representative: Schoppe, Fritz

(56) References cited:
- WO-A-00/44426
- US-A- 3 046 983
- US-A- 4 907 583
- US-A- 6 102 036

## Description

### BACKGROUND OF THE INVENTION

The present invention relates to an inhalator suitable for administering a powder or powder composition, and a powder composition containing powders different in particle diameter from each other and a process for administering the powder composition using inhalators.

Generally, a powder inhalator is used for inhaling a powder or powder composition such as a powdered medicine into a human body through the oral or nasal cavity. The inhalator includes an inhalator body having an air intake path for introducing an ambient air and a suction opening through which an air-powder mixture within the inhalator body is sucked into the oral or nasal cavity. A powder receiving chamber for receiving the powder is disposed within the inhalator body and communicated with the outside of the inhalator body via the air intake path. An air-powder mixture path extends from the powder receiving chamber to the suction opening. The air-powder mixture is formed when the air is introduced into the powder receiving chamber through the air intake path. The air-powder mixture is then transmitted from the powder receiving chamber to the suction opening via the air-powder mixture path.

There are several types of powders different in aerodynamic mean particle diameter as follows: a powder having the aerodynamic mean particle diameter of not less than 7 µm and depositing in an oral cavity or hypoglottis, a powder having the aerodynamic mean particle diameter of 5-7 µm and depositing in a throat, a powder having the aerodynamic mean particle diameter of 3-5 µm and depositing in a trachea, a powder having the aerodynamic mean particle diameter of 1-3 µm and depositing in bronchi, and a powder having the aerodynamic mean particle diameter of not more than 1 µm and depositing into alveoli, and the like. The powder having the aerodynamic mean particle diameter of not more than 3 µm is required to surely reach affected areas of the human body. Also, the powder such as an acrid powder is preferably dosed in several parts upon being inhaled.

In addition, there has been proposed powder tobacco for use with the inhalator. The powder tobacco can be substituted for a usual smoking tobacco because the powder tobacco provides a smoking feeling upon being inhaled. When the powder tobacco is used, one dose of the powder tobacco is dispensed in parts from the inhalator upon each inhalation.

The human bronchi and alveoli exist in deeper portions of the human body. Therefore, in order to ensure stable deposit of the powder having the particle diameter of not more than 3 µm in the bronchi and alveoli, it is preferable to dose the powder in parts, i.e., dispense a small amount of the powder each inhalation.

However, in the earlier technique, the whole amount of the powder received within the powder receiving chamber of the inhalator is dispensed from the inhalator by the inhalation substantially at one time. If a dose of the powder having the particle diameter of not more than 3 µm is inhaled through the inhalator upon inhalation, a large amount of the powder dosed will be deposited in the oral cavity or trachea before being deposited in the bronchi and alveoli.

Further, there is known a process for administering a particulate medicament having a specific mean particle diameter into a patient's lungs upon the patient breathing. International Publication No. WO97/36574 discloses a process and device for inhalation of particulate medicament. The process includes (i) providing an inhalator which contains at least one dose of medicament particles comprising spherical hollow particulates of respirable particle size suitable for deposition in a human lungs, and (ii) removing the spherical hollow particulates from the inhalator. In the earlier technique, the particulate medicament having the specific particle diameter is used with the inhalator, but there is not described inhalation on multi-purpose prescription, for instance, one-time inhalation of multiple particulate medicaments for the purpose of simultaneous deposition in different portions such as the trachea and the alveoli of the patient's body. In order to follow the multi-purpose prescription, it is required that the patient repeatedly inhales separate doses of particulate medicaments for different prescriptions, takes a specific particulate medicament formulated for the multi-purpose prescription, or is treated with the combination of various prescriptions including peroral medicament, injection, application of fomentation, and the like. The WO 00/44426, considered to be the closest prior art for the present invention discloses an inhalation type drug dispenser, comprising a drug dispenser main body having a cylindrical body fitting hole, an inhalation port, inlet side ventilating paths, outlet side ventilation paths, and others and a drug powder storing cylindrical body which is fitted into the cylindrical body fitting hole and opens and closes the ventilating paths with respect to the drug powder storing chamber, whereby the inhalation type drug dispenser can be simplified in structure and reduced in production costs because it is formed of two members of drug dispenser main body and drug powder storing cylindrical body and can prevent drug powder from flowing out because it can enclose the drug powder in the drug powder storing chamber with the drug powder storing cylindrical body.

The US-A-3046983 discloses and inhaler for dispensing volatile medicaments which comprises a tubular elongated barrel having at one end an air inlet port and at the other end an air outlet port, a second air inlet port between the first air inlet port and air outlet port and an absorbent body adapted to hold a volatile medicament within said barrel positioned to the first air inlet port. Furthermore, the inhaler comprises a tubular outer casing covering said tubular barrel closely fitting thereover but being adapted to turn, an air inlet port at one end of said casing positioned so as to overlap the first air inlet port of said barrel, a second air inlet port in said casing so positioned as to overlap in the intermediate air inlet port, an air outlet port in said casing permitting air to flow from the air outlet port of said barrel through the air outlet port of said casing. Herein, the two air inlet ports of said casing and said barrel are in such relationship that as the casing is turned with respect to the barrel overlapping of one set of ports is increased while the overlapping of the second set of air inlet ports is decreased.

US-A-6,102,036 discloses a method of assisting a person to withdraw from cigarette induced nicotine dependency, comprising introducing a predetermined dose of a non-pressurized, particulate medicament comprising at least one nicotine formulation suitable for absorption into the bloodstream of the person through the alveoli and small airways of the person's lungs into a breath activated inhaler for use by said person as a cigarette substitute. A breath activated inhaler which may be used in accordance with this method is also disclosed.

US-A-4,907,583 discloses a device for a powder inhalator intended for inhalation of an air flow which contains pharmacologically active compound in micronized form. The powder inhalator comprises a nozzle unit with a nozzle aperture as well as a container unit with a releasing or dosing unit for delivering the active compound. The air flow generated by inhalation is at least partly aspirated through an air conduit located in the container unit, which conduit extends from an air inlet, communicating with the environment, via said releasing or dosing unit, up to said nozzle unit. Deflector devices are stationarily arranged in the container unit and/or in the nozzle unit, said deflector devices, for example in the shape of a helical channel portion, being arranged to create a powerful deflecting movement for the purpose of disrupting said powder particles into the respirable particle size distribution.

It is the object of the present invention to provide an improved inhalator capable of dispensing a powder or a powder composition deep into the human body with reduced waste of powder and a simplified structure.

This object is achieved by an inhalator according to claim 1.

According to one aspect of the present invention, there is provided an inhalator for administering an air-powder mixture, comprising:
an inhalator body including an air intake path for introducing air into the inhalator body, and an air-powder mixture outlet for discharging the air-powder mixture from the inhalator body;
a powder receiving chamber adapted to receive a powder, the powder receiving chamber being disposed within the inhalator body and communicated with an outside of the inhalator body through the air intake path;
an air-powder mixture path adapted to transmit the air-powder mixture flowing from the powder receiving chamber to the air-powder mixture outlet;
an air-powder mixture reservoir adapted to temporarily store the air-powder mixture flowing from the powder receiving chamber, the air-powder mixture reservoir being disposed within the air-powder mixture path; and
a diluent air passage adapted to introduce a diluent air into the air-powder mixture reservoir, the diluent air passage communicating the air-powder mixture reservoir with the outside of the inhalator body.

According to a further aspect of the present invention, there is provided an inhalator for administering an air-powder mixture, comprising:
a casing including an air intake inlet for introducing air into the casing, and an air-powder mixture outlet for discharging the air-powder mixture from the casing;
powder receiving means for receiving a powder within the casing and permitting the powder to be admixed with the air introduced from the air intake inlet;
air-powder mixture storing means for temporarily storing the air-powder mixture passing through the powder receiving means;
diluent air passage means for permitting a diluent air to flow into the air-powder mixture storing means; and
air-powder mixture path means for permitting the air-powder mixture to flow from the powder receiving means to the air-powder mixture outlet via the air-powder mixture storing means.

According to another aspect of the present invention, there is provided a powder composition for use with an inhalator, comprising:
at least two kinds of fine particles selected from a first kind of fine particles having an aerodynamic mean particle diameter of not less than 7 µm, a second kind of fine particles having an aerodynamic mean particle diameter of 5-7 µm, a third kind of fine particles having an aerodynamic mean particle diameter of 3-5 µm, a fourth kind of fine particles having an aerodynamic mean particle diameter of 1-3 µm, and a fifth kind of fine particles having an aerodynamic mean particle diameter of not more than 1 µm.

According to a further aspect of the present invention, there is provided a process for administering a powder composition using an inhalator, comprising:
preparing the powder composition containing at least two kinds of fine particles selected from a first kind of fine particles having an aerodynamic mean particle diameter of not less than 7 µm, a second kind of fine particles having an aerodynamic mean particle diameter of 5-7 µm, a third kind of fine particles having an aerodynamic mean particle diameter of 3-5 µm, a fourth kind of fine particles having an aerodynamic mean particle diameter of 1-3 µm, and a fifth kind of fine particles having an aerodynamic mean particle diameter of not more than 1 µm;
supplying the powder composition to the inhalator; and
discharging the powder composition from the inhalator.

The other objects and features of this invention will become understood from the following description with reference to the accompanying drawings.

### BRIEF DESCRIPTION OF THE DRAWINGS

Fig. 1 is an elevation of an inhalator of a preferred embodiment, according to the present invention;
Fig. 2 is a longitudinal section of the inhalator, taken along the line 2-2 of Fig. 1, showing a rest position of the inhalator;
Fig. 3 is an enlarged section of the inhalator, taken along the line 3-3 of Fig. 2;
Fig. 4 is an enlarged section of the inhalator, taken along the line 4-4 of Fig. 2;
Fig. 5 is an enlarged section of the inhalator, taken along the line 5-5 of Fig. 2; and
Fig. 6 is a view similar to Fig. 2, but showing a use position of the inhalator.

### DETAILED DESCRIPTION OF THE INVENTION

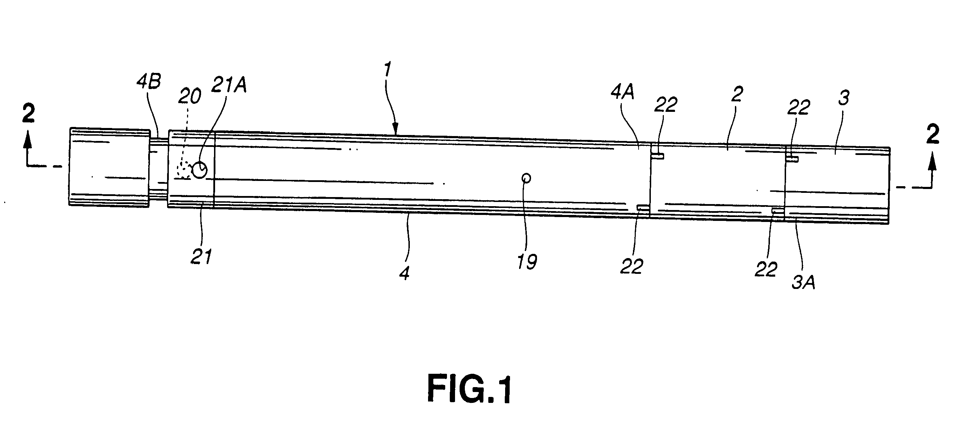

Referring to Figs. 1-6, an inhalator, according to the present invention, of a preferred embodiment is explained.

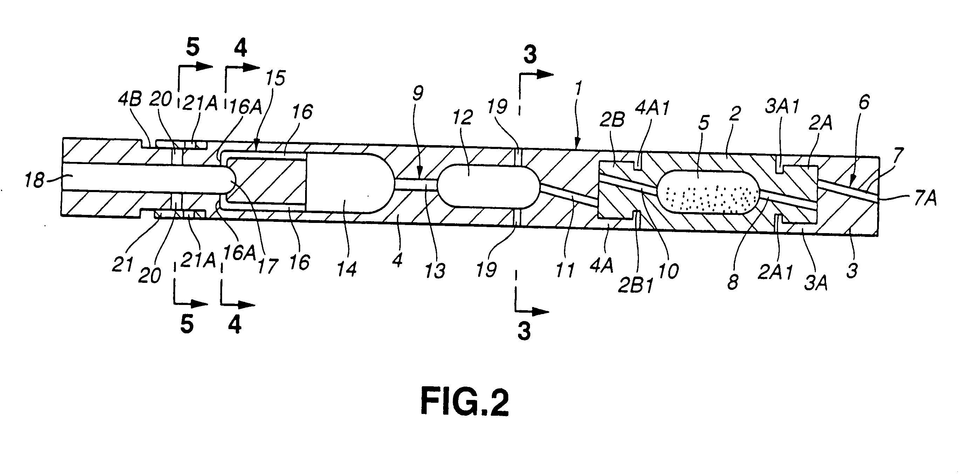

As illustrated in Fig. 1, the inhalator includes an inhalator body 1 as a casing which is formed into a cylindrical shape. Inhalator body 1 is made of a suitable resin material such as polypropylene, polystyrene, ABS resin and the like. Inhalator body 1 is constituted of cap 3, suction body 4 and capsule body 2 interposed between cap 3 and suction body 4. Cap 3 has air intake inlet 7A shown in Fig. 2, through which an ambient air is introduced into cap 3 and flows toward capsule body 2 and suction body 4 as explained later. Cap 3 has a length shorter than that of capsule body 2 and is rotatably connected with an upstream side of capsule body 2. Suction body 4 has air-powder mixture outlet 18 shown in Fig. 2, from which an air-powder mixture formed in inhalator body 1 is discharged into a user's oral cavity. Suction body 4 has a length longer than that of capsule body 2 and is rotatably connected with a downstream side of capsule body 2.

As illustrated in Fig. 2, capsule body 2 has engaging projection 2A and engaging groove 2A1 on the upstream end portion. Engaging projection 2A and engaging groove 2A1 axially adjacent thereto are engaged with engaging tube portion 3A and engaging projection 3A1 of cap 3, respectively. Capsule body 2 is coupled with cap 3 by the engagement of engaging projection 2A and engaging groove 2A1 with engaging tube portion 3A and engaging projection 3A1, respectively. Capsule body 2 also has at the downstream end portion, engaging projection 2B and engaging groove 2B1 axially adjacent thereto. Engaging projection 2B and engaging groove 2B1 are engaged with engaging tube portion 4A and engaging projection 4A1 of suction body 4, respectively. Capsule body 2 is coupled with suction body 4 by the engagement of engaging projection 2B and engaging groove 2B1 with engaging tube portion 4A and engaging projection 4A1, respectively.

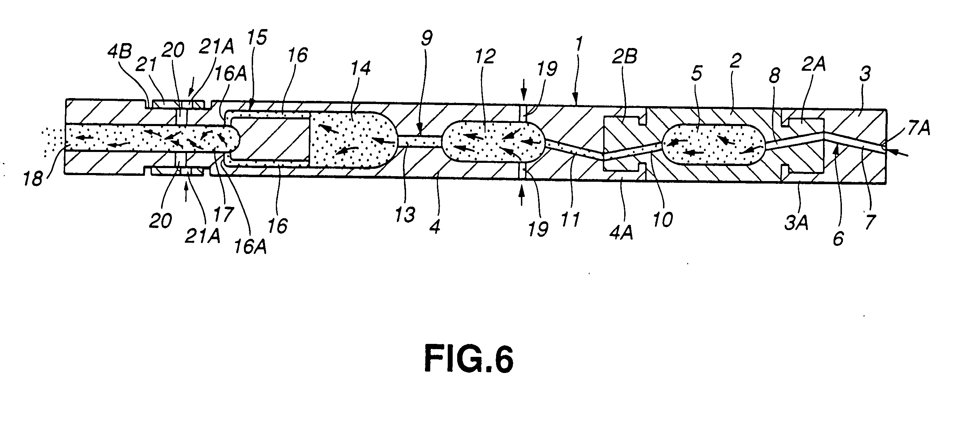

Cocoon-shaped powder receiving chamber 5 is substantially coaxially disposed within capsule body 2. Powder receiving chamber 5 is provided for receiving a dose of a powder or powder composition such as particulate medicament, powder tobacco or the like. Powder receiving chamber 5 is in communication with the outside of inhalator body 1 through air intake path 6, when the inhalator is in a use position as explained later by referring to Fig. 6. The air-powder mixture is formed within powder receiving chamber 5 when the air flows into powder receiving chamber 5 via air intake path 6 in the use position of the inhalator.

Air intake path 6 is provided for introducing the air into powder receiving chamber 5. Air intake path 6 includes upstream intake passage 7 formed in cap 3 and downstream intake passage 8 formed in capsule body 2. Upstream intake passage 7 has an upstream end opening as air intake inlet 7A which is open to a generally central portion of an axial end face of cap 3. Upstream intake passage 7 has a downstream end opening that is open to a bottom of engaging tube portion 3A which mates with an axial end face of engaging projection 2A, in an offset position relative to the center axis of cap 3. Downstream intake passage 8 has an upstream end opening that is open to the axial end face of engaging projection 2A in an offset position relative to the center axis of capsule body 2. Downstream intake passage 8 has a downstream end opening that is open to an upstream end portion of powder receiving chamber 5 and in substantially alignment with the center axis of capsule body 2.

Capsule body 2 and cap 3 are relatively rotatable to be placed in a non-communication position shown in Fig. 2 and a communication position shown in Fig. 6. In the non-communication position, the downstream end opening of upstream intake passage 7 and the upstream end opening of downstream intake passage 8 are out of alignment with each other. Fluid communication between upstream intake passage 7 and downstream intake passage 8 is blocked so that powder receiving chamber 5 is prevented from being fluidly communicated with the outside of the inhalator. On the other hand, in the communication position, the downstream end opening of upstream intake passage 7 and the upstream end opening of downstream intake passage 8 are in alignment with each other. The fluid communication between upstream intake passage 7 and downstream intake passage 8 is established so that powder receiving chamber 5 is fluidly communicated with the outside of the inhalator. The opening area at the connection of upstream intake passage 7 and downstream intake passage 8 may be desirably regulated by adjusting the alignment of intake passages 7 and 8 to thereby control a flow amount of the air introduced into powder receiving chamber 5 and therefore control an amount of the powder in the air-powder mixture flowing from powder receiving chamber 5 toward air-powder mixture outlet 18.

Air-powder mixture path 9 extends between powder receiving chamber 5 and air-powder mixture outlet 18. Air-powder mixture path 9 permits the air-powder mixture to flow from powder receiving chamber 5 to air-powder mixture outlet 18 when the inhalator is in the use position.

First air-powder mixture reservoir 12 is disposed within air-powder mixture path 9. Air-powder mixture reservoir 12 is disposed in substantially coaxial with suction body 4. Air-powder mixture reservoir 12 is adapted to be communicated with powder receiving chamber 5 through discharge passage 10 and connecting passage 11 of air-powder mixture path 9. Air-powder mixture reservoir 12 has a cocoon shape having a volumetric capacity greater than a volumetric capacity of connecting passage 11. Air-powder mixture reservoir 12 having the greater volumetric capacity allows the air-powder mixture flowing thereinto through connecting passage 11 to be temporarily stored.

Discharge passage 10 is formed in capsule body 2 so as to be open to powder receiving chamber 5 at the upstream end and to engaging projection 2B at the downstream end. An upstream end opening of discharge passage 10 is open to a downstream end portion of powder receiving chamber 5 and in substantially alignment with the center axis of capsule body 2. A downstream end opening of discharge passage 10 is open to an axial end face of engaging projection 2B in an offset position relative to the center axis of capsule body 2. Connecting passage 11 is formed in suction body 4 so as to be open to engaging tube portion 4A at the upstream end and to air-powder mixture reservoir 12 at the downstream end. An upstream end opening of connecting passage 11 is open to a bottom of engaging tube portion 4A which mates with the axial end face of engaging projection 2B, in an offset position relative to the center axis of suction body 4. A downstream end opening of connecting passage 11 is open to an upstream end portion of air-powder mixture reservoir 12 and in substantially alignment with the center axis of suction body 4. Capsule body 2 and suction body 4 are relatively rotatable so as to be placed in a non-communication position shown in Fig. 2 and a communication position shown in Fig. 6. In the non-communication position, the downstream end opening of discharge passage 10 and the upstream end opening of connecting passage 11 are out of alignment with each other so that fluid communication between discharge passage 10 and connecting passage 11 is blocked. Powder receiving chamber 5 is prevented from being fluidly communicated with air-powder mixture reservoir 12. On the contrary, in the communication position, the downstream end opening of discharge passage 10 and the upstream end opening of connecting passage 11 are in alignment with each other. The fluid communication between discharge passage 10 and connecting passage 11 is established so that powder receiving chamber 5 is fluidly communicated with the air-powder mixture reservoir 12. The opening area at the connection of discharge passage 10 and connecting passage 11 may be desirably regulated by adjusting the alignment of discharge passage 10 and connecting passage 11 to thereby control an amount of the air-powder mixture flowing from powder receiving chamber 5 into air-powder mixture reservoir 12.

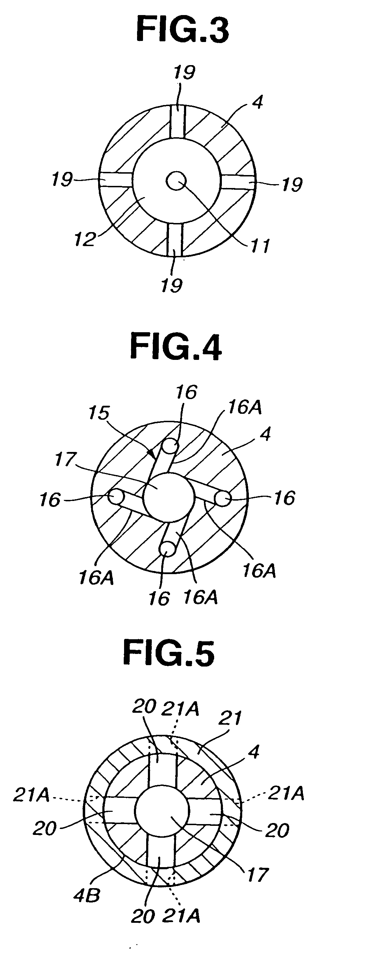

First diluent air passage 19 is formed in suction body 4 and communicated with air-powder mixture reservoir 12. Diluent air passage 19 introduces a diluent air into air-powder mixture reservoir 12 when the air-powder mixture flows from powder receiving chamber 5 into air-powder mixture reservoir 12. The diluent air introduced is merged in the air-powder mixture within air-powder mixture reservoir 12 to thereby dilute the air-powder mixture. The diluted air-powder mixture flowing from air-powder mixture reservoir 12 has a reduced flow rate and a decreased mixing ratio of the powder relative to the air which are present in the diluted air-powder mixture. Diluent air passage 19 is constituted of four passages arranged in crossed manner in lateral section in this embodiment as shown in Fig. 3. As illustrated in Fig. 3, each of four diluent air passages 19 has an inlet open to an outer circumferential surface of suction body 4 and an outlet open to a circumferential surface of air-powder mixture reservoir 12.

Second air-powder mixture reservoir 14 is disposed within air-powder mixture path 9 downstream of first air-powder mixture reservoir 12. Air-powder mixture reservoir 14 is disposed in substantially coaxial relation to suction body 4. Air-powder mixture reservoir 14 is communicated with air-powder mixture reservoir 12 through communication passage 13 of connecting passage 11 which extends in the axial direction of suction body 4. Air-powder mixture reservoir 14 has a bell shape having a volumetric capacity greater than a volumetric capacity of communication passage 13 when viewed in axial cross-section. Air-powder mixture reservoir 14 with the greater volumetric capacity allows the air-powder mixture flowing thereinto through communication passage 13 to be temporarily stored.

Dispersion part 15 is disposed within air-powder mixture path 9 downstream of second air-powder mixture reservoir 14. Dispersion part 15 is adapted to prevent the powder in the air-powder mixture flowing from second air-powder mixture reservoir 14 from aggregating together and intimately mix the powder and the air to form a uniform air-powder mixture. Dispersion part 15 includes dispersion chamber 17 and a plurality of dispersion passages 16 connected with dispersion chamber 17. Dispersion passages 16, four passages in this embodiment, connect dispersion chamber 17 with air-powder mixture reservoir 14. Each of dispersion passages 16 has an inlet open to air-powder mixture reservoir 14 and an outlet open to dispersion chamber 17. Specifically, dispersion passage 16 includes an inlet passage portion extending from an outer peripheral portion of air-powder mixture reservoir 14 in the axial direction of suction body 4. Dispersion passage 16 also includes outlet passage portion 16A that radially inwardly extends from a downstream side of the inlet passage portion and is open to an upstream end portion of dispersion chamber 17. As illustrated in Fig. 4, dispersion chamber 17 has a generally circular-shaped section and outlet passage portion 16A extends in a tangential direction of dispersion chamber 17. The air-powder mixture flowing into dispersion chamber 17 through dispersion passages 16 forms a swirl flow within dispersion chamber 17. The swirl flow of the air-powder mixture prevents the powder in the air-powder mixture from forming an aggregated mass of the powder.

Second diluent air passage 20 is formed within suction body 4 in communication with dispersion chamber 17. As seen from Figs. 2 and 5, four diluent air passages 20 radially extend from grooved portion 4B on an outer surface of suction body 4 to dispersion chamber 17. Grooved portion 4B extends along the entire circumference of the outer surface of suction body 4. Diluent air passages 20 introduce the ambient air as a diluent air into dispersion chamber 17 when the air-powder mixture within dispersion chamber 17 is directed toward outlet 18 by the user's suction.

Regulator 21 for variably controlling a flow amount of the diluent air introduced into dispersion chamber 17 via diluent air passages 20 is axially moveably disposed on grooved portion 4B of suction body 4. Regulator 21 is in the form of a ring in this embodiment. Regulator 21 has four regulator holes 21A coming into alignment with diluent air passages 20 by the axial movement of the regulator 21. Regulator 21 variably regulates an opening area of each of diluent air passages 20 to thereby variably control the flow amount of the diluent air which is merged in the air-powder mixture within dispersion chamber 17.

The air-powder mixture passing through dispersion passages 16 and dispersion chamber 17 flows to air-powder mixture outlet 18 from which the air-powder mixture is dispensed into the user's oral cavity. Air-powder mixture outlet 18 is communicated with dispersion chamber 17 and open to one axial end surface of suction body 4. Air-powder mixture outlet 18 is disposed substantially coaxially with the center axis of suction body 4.

Referring back to Fig. 1, counter or registration marks 22, 22, 22 are formed on the upstream and downstream end portions of the outer circumferential surface of capsule body 2, downstream engaging tube portion 3A of cap 3, and upstream engaging tube portion 4A of suction body 4, respectively. When counter mark 22 on the upstream-end side of capsule body 2 is aligned with counter mark 22 on the downstream-end side of cap 3, upstream and downstream intake passages 7 and 8 of air intake path 6 are communicated with each other. When counter mark 22 on the downstream-end side of capsule body 2 is aligned with counter mark 22 on the upstream-end side of suction body 4, discharge passage 10 and connecting passage 11 of air-powder mixture path 9 are communicated with each other.

An operation of the thus-constructed inhalator of the present invention will be explained hereinafter.

When the inhalator is in a rest or nonuse position shown in Fig. 2, upstream and downstream intake passages 7 and 8 of air intake path 6 are fluidly disconnected from each other and discharge passage 10 and connecting passage 11 of air-powder mixture path 9 are fluidly disconnected from each other. In this state, powder receiving chamber 5 is prevented from being fluidly communicated with the outside of inhalator body 1 and air-powder mixture reservoir 12. Thus, if the inhalator is in the rest position, the powder received within powder receiving chamber 5 can be restrained from flowing therefrom and inhalator body 1 when the user carries the inhalator.

Next, upon using the inhalator, cap 3 and suction body 4 are rotated relative to capsule body 2 to align respective counter marks 22 with each other. Regulator 21 is axially moved in grooved portion 4B so as to desirably adjust the opening area of second diluent air passage 20. The inhalator is thus placed in a use position shown in Fig. 6. In the use position, upstream and downstream intake passages 7 and 8 of air intake path 6 are fluidly connected with each other and discharge passage 10 and connecting passage 11 of air-powder mixture path 9 are fluidly connected with each other. Powder receiving chamber 5 is allowed to be in fluid communication with the outside of inhalator body 1 and air-powder mixture reservoir 12. In this state, air-powder mixture outlet 18 of inhalator body 1 is put into the user's oral cavity and the ambient air is sucked by the user. The air is introduced into air intake path 6 through air intake inlet 7A. The air then flows into powder receiving chamber 5 as indicated by arrows in Fig. 6. The introduced air is admixed with the dose of the powder within powder receiving chamber 5, forming the air-powder mixture. The air-powder mixture flows into first air-powder mixture reservoir 12 via discharge passage 10 and connecting passage 11 of air-powder mixture path 9. The air-powder mixture is temporarily stored within air-powder mixture reservoir 12 and admixed with the diluent air introduced through diluent air passage 19. The thus diluted air-powder mixture has a decreased flow rate flowing into communication passage 13, and a reduced mixing ratio of the powder in the diluted air-powder mixture to the air in the diluted air-powder mixture.

The diluted air-powder mixture within first air-powder mixture reservoir 12 flows into second air-powder mixture reservoir 14 via communication passage 13 and then enters into dispersion chamber 17 via dispersion passages 16. There occurs a swirl flow of the diluted air-powder mixture within dispersion chamber 17. The swirl flow atomizes an aggregated mass of the powder which remains in dispersion chamber 17, to thereby assure the air-powder mixture containing fine particles of the powder in a suitably dispersed state. The air-powder mixture within dispersion chamber 17 is diluted by the diluent air introduced thereinto through second diluent air passage 20 and regulator holes 21A of regulator 21. The thus diluted air-powder mixture then is discharged from air-powder mixture outlet 18 into the user's oral cavity.

As be appreciated from the above explanation, the air-powder mixture flowing from powder receiving chamber 5 is diluted within air-powder mixture reservoir 12 by the diluent air introduced into air-powder mixture reservoir 12 through diluent air passage 19. A flow rate of the air-powder mixture is reduced within air-powder mixture reservoir 12 by the introduction of the diluent air. As a result, a part of the dose of the powder received within powder receiving chamber 5 is sucked by one-time inhalation by the user. Therefore, the dose of the powder received within powder receiving chamber 5 can be divided into a plurality of dose parts each being sucked by the user. Thus, the user can suck a small amount of the powder that forms each dose part, by one-time inhalation. If it is required to deposit fine particulate medicament having a small particle diameter in the bronchi or alveoli of a patient, a dose of the medicament can be dispensed in parts which are inhaled by multiple-time inhalation of the user through the inhalator of the invention. The fine particulate medicament can be prevented from being deposited in the trachea and be stably deposited in the bronchi or alveoli by multiple-time inhalation of the dose parts. The inhalator of the invention can be effectively used for dispensing a dose of a powder or powder composition such as particulate medicament and powder tobacco, in parts by multiple-time inhalation.

Further, with the arrangement of second diluent air passage 20 and regulator 21 for regulating the opening area of diluent air passage 20, an amount of the diluent air introduced into dispersion chamber 17 can be desirably regulated by axially moving regulator 21. A mixing ratio between the powder and the air present in the air-powder mixture within dispersion chamber 17 can be readily controlled by the regulation of the diluent air to be introduced. Accordingly, an amount of the powder which is sucked by one-time inhalation by the user, can be desirably controlled using regulator 21 depending on the user's liking, kinds of particulate medicaments, or the like. This can improve a performance of the inhalator. The amount of the powder for one-time inhalation may be controlled by regulating the opening area at the connection of upstream and downstream intake passages 7 and 8 of air intake path 6 or the opening area at the connection of discharge passage 10 and connecting passage 11 of air-powder mixture path 9.

Furthermore, with the arrangement of dispersion passages 16 and dispersion chamber 17 at dispersion part 15, the swirl flow of the air-powder mixture can be produced within dispersion chamber 17, which atomizes an aggregated mass of the powder remaining in dispersion chamber 17 and forms the air-powder mixture containing the powder particles in a good dispersed state. This can improve a dispersion efficiency of the inhalator.

Further, upstream and downstream intake passages 7 and 8 of air intake path 6 is arranged to establish and block the fluid communication between powder receiving chamber 5 and the outside of inhalator body 1. When the inhalator is in the nonuse position, upstream and downstream intake passages 7 and 8 are disconnected from each other so that the fluid communication between powder receiving chamber 5 and the outside of inhalator body 1 is blocked. In addition, discharge passage 10 and connecting passage 11 of air-powder mixture path 9 is arranged to allow and block the fluid communication between powder receiving chamber 5 and first air-powder mixture reservoir 12. In the nonuse position of the inhalator, discharge passage 10 and connecting passage 11 are disconnected from each other so that the fluid communication between powder receiving chamber 5 and first air-powder mixture reservoir 12 is blocked. With this arrangement of intake passages 7 and 8 and discharge passage 10 and connecting passage 11, the powder received within powder receiving chamber 5 can be prevented from flowing therefrom toward both air intake inlet 7A and air-powder mixture reservoir 12 upon the user carrying the inhalator. This can improve reliability of the inhalator. Further, when intake passages 7 and 8 are communicated with each other upon using the inhalator, the opening area of the connection of intake passages 7 and 8 can be regulated to control the flow amount of the air flowing into powder receiving chamber 5. Therefore, the amount of the powder present in the air-powder mixture produced within powder receiving chamber 5 can be adjusted. Similarly, upon communication of discharge passage 10 and connecting passage 11, the opening area of the connection thereof can be regulated to control the flow amount of the air-powder mixture flowing from powder receiving chamber 5 into air-powder mixture reservoir 12. The amount of the powder in the air-powder mixture flowing from air-powder mixture reservoir 12 toward air-powder mixture outlet 18 can be adjusted, and therefore, the amount of the powder to be sucked can be adjusted.

Although two air-powder mixture reservoirs 12 and 14 are provided within suction body 4 in this embodiment, a single air-powder mixture reservoir or three or more air-powder mixture reservoirs may be provided.

In addition, a capsule chamber for storing a capsule having a dose of the powder may be substituted for powder receiving chamber 5. In this case, the capsule within the capsule chamber may be pierced using a piercing device upon inhalation.

Further, a shutter member may be provided for blocking and allowing the fluid communication between powder receiving chamber 5 and the outside of inhalator body 1 and air-powder mixture reservoir 12, instead of the arrangement of upstream and downstream intake passages 7 and 8 of air intake path 6 and discharge passage 10 and connecting passage 11 of air-powder mixture path 9. The shutter member may be rotatably or slidably disposed within air intake path 6 extending between powder receiving chamber 5 and air intake inlet 7A and the portion of air-powder mixture path 9 which extends between powder receiving chamber 5 and air-powder mixture reservoir 12.

Furthermore, either one of the upstream end portion of capsule body 2 and engaging tube portion 3A of cap 3 may have on the outer circumferential surface a groove circumferentially extending within a predetermined angular region. The other may have on the outer circumferential surface a projection engageable with the groove such that both capsule body 2 and cap 3 are rotatably moveable to each other in the predetermined angular region. A similar circumferentially extending groove may be formed on either one of the outer circumferential surface of the downstream end portion of capsule body 2 and the outer circumferential surface of engaging tube portion 4A of suction body 4, and a similar projection may be formed on the other thereof. If the projections reach the respective ends of the grooves, the communication between upstream and downstream intake passages 7 and 8 and the communication between discharge passage 10 and connecting passage 11 will be established. In this case, counter marks 22 can be omitted.

Next, a powder composition for use with inhalators and a process for administering the powder composition using inhalators, according to the present invention, will be explained hereinafter.

The powder composition is suitable to be administered from an oral or nasal cavity for deposition in inside parts of the human body. The powder composition includes at least two kinds of fine particles selected from a group consisting of a first kind of fine particle having an aerodynamic mean particle diameter of not less than 7 µm, a second kind of fine particle having an aerodynamic mean particle diameter of 5-7 µm, a third kind of fine particle having an aerodynamic mean particle diameter of 3-5 µm, a fourth kind of fine particle having an aerodynamic mean particle diameter of 1-3 µm, and a fifth kind of fine particle having an aerodynamic mean particle diameter of not more than 1 µm. The first kind of fine particle having the aerodynamic mean particle diameter of not less than 7 µm is deposited in an oral cavity or hypoglottis of a human body. The second kind of fine particle having the aerodynamic mean particle diameter of 5-7 µm is deposited in a throat of a human body. The third kind of fine particle having the aerodynamic mean particle diameter of 3-5 µm is deposited in a trachea of a human body. The fourth kind of fine particle having the aerodynamic mean particle diameter of 1-3 µm is deposited in bronchi of a human body. The fifth kind of fine particle having the aerodynamic mean particle diameter of not more than 1 µm is deposited in alveoli of a human body.

Preferably, the fine particles of the powder composition of the present invention have a significantly narrow particle size distribution. More preferably, the fine particles have the particle size distribution consistent with a predetermined range of an aerodynamic mean particle diameter which is required for deposition in the respective parts of the human body.

The powder composition may be powder tobacco and particulate medicament. The powder tobacco contains at least two kinds of fine particles selected from the first, third and fifth kinds of fine particles as described above. For instance, the powder tobacco may contain fine particles as a gustatory component which have the aerodynamic mean particle diameter of 45-55 µm for deposition in the oral cavity or hypoglottis, fine particles as a stimulatory component which have the aerodynamic mean particle diameter of 3-5 µm for deposition in the trachea or throat, and fine particles as an agent which have the aerodynamic mean particle diameter of 0.5-2 µm for deposition in the alveoli or bronchi. A coffee extract powder may be used for the fine particles as a gustatory component having the aerodynamic mean particle diameter of 45-55 µm. A menthol extract powder may be used for the fine particles as a stimulatory component having the aerodynamic mean particle diameter of 3-5 µm. A nicotine extract powder may be used for the fine particles as an agent having the aerodynamic mean particle diameter of 0.5-2 µm. If the powder tobacco is inhaled with the inhalator, the same taste, stimulus and nicotinic effect as those obtained by smoking can be obtained.

The particulate medicament as the powder composition of the present invention contains at least two kinds of fine particles selected from the first through fifth kinds of fine particles as described above. The particulate medicament may contain fine particles as a gustatory component which have the aerodynamic mean particle diameter of 60-80 µm for deposition in the oral cavity or hypoglottis, fine particles as an antiphlogistic agent which have the aerodynamic mean particle diameter of 4-6 µm for deposition in the trachea or throat, and fine particles as an agent which have the aerodynamic mean particle diameter of 1-3 µm for deposition in the alveoli or bronchi. A powdered troche or candy may be used for the fine particles as a gustatory component having the aerodynamic mean particle diameter of 60-80 µm. An antiphlogistic powder may be used for the fine particles as an antiphlogistic agent having the aerodynamic mean particle diameter of 4-6 µm. An antibiotic powder may be used for the fine particles as an agent having the aerodynamic mean particle diameter of 1-3 µm.

In addition, the particulate medicament as the powder composition of the present invention may be selected from an analgesic agent, an anginal preparation, an antiallergic agent, an anti-infective agent, an antihistaminic agent, an anti-inflammatory agent, an antitussive agent, a bronchodilator agent, a diuretic agent, an anticholinergic agent, and the like, depending on cure purposes. These powder agents may have various aerodynamic mean particle diameters suitable for deposition in different target parts of the human body.

If required, the particulate medicament as the powder composition of the present invention may be used together with a known excipient acceptable for inhalation into the human body. The composition of the particulate medicament is prepared in accordance with the doctor's prescription given on the basis of the patient's symptom.

In the administration process of the present invention, first the powder composition is prepared so as to contain at least two kinds of fine particles selected from the first to fifth kinds of fine particles as described above. The at least two kinds of fine particles of the powder composition may be blended together. The thus prepared powder composition is supplied to an inhalator suitable for dispensing a powder into the human body. The powder composition may be capsulated and then accommodated in the inhalator. Subsequently, the powder composition supplied is discharged from the inhalator. If the above-described inhalator of the present invention is used, the powder composition may be dispersed within the inhalator and then discharged therefrom without aggregation of the fine particles of the powder composition.

The powder composition and administration process of the present invention can be suitably used for cure of multiple diseases using the particulate medicaments which have effects on the multiple diseases, respectively. Specially, the powder composition and administration process of the present invention is suitable for providing analgesia and curing inflammation in the oral cavity and/or throat, asthma, bronchitis, COPD (chronic obstructive pulmonary disease), respiratory disease such as thoracho-infection, and allergosis.

The inhalators useable in this embodiment are described in Japanese Patent Applications First Publication Nos. 62-41668 and 9-47509, Japanese Patent Application Second Publication No. 63-6024, and United States Patent No. 5,996,577.

### EXAMPLES

The present invention is described in more detail by way of examples. However, these examples are only illustrative and not intended to limit a scope of the present invention thereto.

### Example 1

A dose of a powder tobacco was prepared by blending 5 mg of coffee extract particulates having an aerodynamic mean particle diameter of 50 µm, 10 mg of menthol extract particulates having an aerodynamic mean particle diameter of 4 µm, and 1 mg of nicotine extract particulates having an aerodynamic mean particle diameter of 0.5-2 µm together. The thus prepared dose of a powder tobacco was supplied to a suitable inhalator as described above and then discharged from the inhalator.

### Example 2

A dose of a particulate medicament mixture was prepared by blending candy particles having an aerodynamic mean particle diameter of 70 µm, antiphlogistic agent particles having an aerodynamic mean particle diameter of 5 µm, antibiotic agent particles having an aerodynamic mean particle diameter of 2 µm together in accordance with a doctor's prescription. The thus prepared dose of a particulate medicament mixture was filled in a capsule. The thus capsulated dose of a particulate medicament mixture was accommodated in a suitable inhalator as described above and then discharged from the inhalator.

Using the powder composition and the administration process of the present invention, a dose of the powder composition containing the at least two kinds of fine particulates different in mean particle diameter from each other can be selected depending on the target parts of the human body in which the powder composition is required to be deposited, and can be deposited in the target parts by one-time inhalation using the inhalator. Namely, multi-purpose dosage of particulate medicaments, for instance, deposition of the particulate medicaments in both of the trachea and the alveoli or all of the throat, the bronchi and the alveoli, can be achieved during the one-time inhalation.

Further, using the powder composition and the administration process of the present invention, the patient can dispense with multiple times of inhalation for dosing a plurality of particulate medicaments required in different prescriptions. Also, any specific compound of particulate medicaments may not be required for multi-purpose prescription.

Furthermore, in a case where the capsulated powder composition of particulate medicaments having different mean particle diameters is used, the patient can dispense with adjusting the amount of the powder composition required for each inhalation and the mixing ratio of the different kinds of particulate medicaments.

Although the invention has been described above by reference to certain embodiments of the invention, the invention is not limited to the embodiments described above. Modifications and variations of the embodiment described above will occur to those skilled in the art, in light of the above teachings. The scope of the invention is defined with reference to the following claims.

## Claims

1. An inhalator for administering an air-powder mixture, comprising:
an inhalator body (1) including an air intake path (6) for introducing an air into the inhalator body (1), and an air-powder mixture outlet (18) for discharging the air-powder mixture from the inhalator body (1);
a powder receiving chamber (5) adapted to receive a powder, the powder receiving chamber (5) being disposed within the inhalator body (1) and communicated with an outside of the inhalator body (1) through the air intake path (6);
an air-powder mixture path (9) adapted to transmit the air-powder mixture flowing from the powder receiving chamber to the air-powder mixture outlet(18); and
a dispersion part (15) adapted to disperse the powder in the air-powder mixture passing through the air-powder mixture path (9);
**characterized by**
an air-powder mixture reservoir (12) disposed within the air-powder mixture path (9) between the powder receiving chamber (5) and the dispersion part (15), the air-powder mixture reservoir (12) adapted to be in communication with the powder receiving chamber (5) and the outside of the inhalator body (1), thereby receiving a part of a dose of the powder within the powder receiving chamber (5) by a one-time inhalation of the user; and
a diluent air passage (19) for providing the communication of the air-powder mixture reservoir (12) with the outside of the inhalator body (1) and adapted to introduce a diluent air into the air-powder mixture reservoir (12),
wherein the dispersion part (15) is adapted to receive from the air-powder mixture reservoir (12) the part of the dose and to provide the dispersed part of the dose to the air-powder mixture outlet (18).

2. The inhalator as claimed in claim 1, further comprising a second diluent air passage (20) adapted to introduce a diluent air into the air-powder mixture path (9) downstream of the air-powder mixture reservoir (12) upon the air-powder mixture flowing from the air-powder mixture reservoir (12), and a regulator (21) variably controlling an opening area of the second diluent air passage (20).

3. The inhalator as claimed in claim 1, wherein the dispersion part (15) comprises a plurality of dispersion passages branched from the air-powder mixture path (9) downstream of the air-powder mixture reservoir (12), and a dispersion chamber (17) disposed within the air-powder mixture path (9) downstream of the dispersion passages, each of the dispersion passages having an outlet passage portion that is open into the dispersion chamber (17) and arranged to form a swirl flow of the air-powder mixture.

4. The inhalator as claimed in claim 3, wherein the dispersion chamber (17) has a generally circular-shaped section and the outlet passage portion of each of the dispersion passages extends in a tangential direction of the dispersion chamber (17).

5. The inhalator as claimed in claim 1, wherein the air intake path (6) is arranged to allow and block fluid communication between the powder receiving chamber (5) and the outside of the inhalator body (1).

6. The inhalator as claimed in claim 5, wherein the air intake path (6) comprises at least two passages having an alignment position where the at least two passages are in alignment with each other and an offset position where the at least two passages are out of alignment with each other.

7. The inhalator as claimed in claim 1, wherein the air-powder mixture path (9) is arranged to allow and block fluid communication between the powder receiving chamber (5) and the air-powder mixture reservoir (12).

8. The inhalator as claimed in claim 7, wherein the air-powder mixture path (9) comprises at least two passages disposed between the powder receiving chamber (5) and the air-powder mixture reservoir (12), the plurality of passages having an alignment position where the at least two passages are aligned with each other and an offset position where the at least two passages are offset from each other.

9. The inhalator as claimed in claim 3, further comprising a second air-powder mixture reservoir (14) adapted to temporarily store the air-powder mixture flowing from the first air-powder mixture reservoir (12) toward the dispersion passages of the dispersion part.

10. The inhalator as claimed in claim 9, wherein each of the dispersion passages comprises an inlet open into the second air-powder mixture reservoir (14).

## Patentansprüche

1. Eine Inhalationsvorrichtung zum Verabreichen einer Luft-Pulver-Mischung, die folgende Merkmale aufweist:
einen Inhalations-Vorrichtungskörper (1), der einen Luftaufnahmeweg (6) zum Einführen einer Luft in den Inhalationsvorrichtungskörper (1) und einen Luft-Pulver-Mischungsauslass (18) zum Entladen der Luft-Pulver-Mischung aus dem Inhalationsvorrichtungskörper (1) umfasst;
eine Pulveraufnahmekammer (5), die angepasst ist, um ein Pulver aufzunehmen, wobei die Pulveraufnahmekammer (5) innerhalb des Inhalationsvorrichtungskörpers (1) angeordnet ist und durch den Luftaufnahmeweg (6) mit einer Außenseite des Inhalationsvorrichtungskörpers (1) in Verbindung steht;
einen Luft-Pulver-Mischungsweg (9), der angepasst ist, um die Luft-Pulver-Mischung, die von der Pulveraufnahmekammer zu dem Luft-Pulver-Mischungsauslass (18) strömt, durchzulassen; und
ein Dispersionsteil (15), das angepasst ist, um das Pulver in der Luft-Pulver-Mischung, die sich durch den Luft-Pulver-Mischungsweg (9) bewegt, zu dispergieren;
**gekennzeichnet durch**
ein Luft-Pulver-Mischungsreservoir (12), das innerhalb des Luft-Pulver-Mischungswegs (9) zwischen der Pulveraufnahmekammer (5) und dem Dispersionsteil (15) angeordnet ist, wobei das Luft-Pulver-Mischungsreservoir (12) angepasst ist, um mit der Pulveraufnahmekammer (5) und der Außenseite des Inhalationsvorrichtungskörpers (1) in Verbindung zu stehen, wodurch es **durch** eine einmalige Inhalation des Benutzers einen Teil einer Dosis des Pulvers innerhalb der Pulveraufnahmekammer (5) aufnimmt; und
einen Verdünnungsmittelluftdurchgang (19) zum Bereitstellen der Verbindung zwischen dem Luft-Pulver-Mischungsreservoir (12) und der Außenseite des Inhalationsvorrichtungskörpers (1), der angepasst ist, um eine Verdünnungsmittelluft in das Luft-Pulver-Mischungsreservoir (12) einzuführen,
wobei das Dispersionsteil (15) angepasst ist, um von dem Luft-Pulver-Mischungsreservoir (12) den Teil der Dosis aufzunehmen und den dispergierten Teil der Dosis dem Luft-Pulver-Mischungsauslass (18) zur Verfügung zu stellen.

2. Die Inhalationsvorrichtung gemäß Anspruch (1), die ferner einen zweiten Verdünnungsmittelluftdurchgang (20), der angepasst ist, um, folgend auf das Strömen der Luft-Pulver-Mischung aus dem Luft-Pulver-Mischungsreservoir (12), dem Luft-Pulver-Mischungsreservoir (12) nachgelagert eine Verdünnungsmittelluft in den Luft-Pulver-Mischungsweg (9) einzuführen, und einen Regler (21), der einen Öffnungsbereich des zweiten Verdünnungsmittelluftdurchgangs (20) einstellbar steuert, aufweist.

3. Die Inhalationsvorrichtung gemäß Anspruch (1), bei der der Dispersionsteil (15) eine Mehrzahl von Dispersionsdurchgängen, die, dem Luft-Pulver-Mischungsreservoir (12) nachgelagert, von dem Luft-Pulver-Mischungsweg (9) abzweigen und eine Dispersionskammer (17), die, den Dispersionsdurchgängen nachgelagert, innerhalb des Luft-Pulver-Mischungs-Wegs (9) angeordnet ist, aufweist, wobei jeder der Dispersionsdurchgänge einen Auslassdurchgangsabschnitt aufweist, der in die Dispersionskammer (17) geöffnet und angeordnet ist, um einen Wirbelstrom der Luft-Pulver-Mischung zu bilden.

4. Die Inhalationsvorrichtung gemäß Anspruch 3, bei der die Dispersionskammer (17) einen in der Regel kreisförmigen Abschnitt aufweist und sich der Auslassdurchganasabschnitt jedes der Dispersionsdurchgänge in einer tangentialen Richtung der Dispersionskammer (17) erstreckt.

5. Die Inhalationsvorrichtung gemäß Anspruch (1), bei der der Luftaufnahmeweg (6) angeordnet ist, um eine Fluidkommunikation zwischen der Pulveraufnahmekammer (5) und der Außenseite des Inhalationsvorrichtungskörpers (1) zuzulassen und zu sperren.

6. Die Inhalationsvorrichtung gemäß Anspruch 5, bei der der Luftaufnahmeweg (6) mindestens zwei Durchgänge aufweist, die eine Ausrichtungsposition, in der die zumindest zwei Durchgänge miteinander ausgerichtet sind, und eine Versatzposition, in der die zumindest zwei Durchgänge nicht miteinander ausgerichtet sind, aufweisen.

7. Die Inhalationsvorrichtung gemäß Anspruch (1), bei der der Luft-Pulver-Mischungsweg (9) angeordnet ist, um eine Fluidkommunikation zwischen der Pulveraufnahmekammer (5) und dem Luft-Pulver-Mischungsreservoir (12) zuzulassen und zu sperren.

8. Die Inhalationsvorrichtung gemäß Anspruch (7), bei der der Luft-Pulver-Mischungsweg (9) zumindest zwei Durchgänge aufweist, die zwischen der Pulveraufnahmekammer (5) und dem Luft-Pulver-Mischungsreservoir (12) angeordnet sind, wobei die Mehrzahl der Durchgänge eine Ausrichtungsposition, in der die zumindest zwei Durchgänge miteinander ausgerichtet sind, und eine Versatzposition, in der die zumindest zwei Durchgänge voneinander versetzt sind, aufweist.

9. Die Inhalationsvorrichtung gemäß Anspruch (3), die ferner ein zweites Luft-Pulver-Mischungsreservoir (14) aufweist, das angepasst ist, um die Luft-Pulver-Mischung, die aus dem ersten Luft-Pulver-Mischungsreservoir (12) zu den Dispersionsdurchgängen des Dispersionsteils strömt, temporär zu speichern.

10. Die Inhalationsvorrichtung gemäß Anspruch (9), bei der jeder der Dispersionsdurchgänge einen Einlass aufweist, der in das zweite Luft-Pulver-Mischungsreservoir (14) geöffnet ist.

## Revendications

1. Inhalateur pour l'administration d'un mélange d'air et de poudre, comprenant :
un corps d'inhalateur (1) comportant un trajet d'entrée d'air (6) pour l'introduction d'air dans le corps d'inhalateur (1), et une sortie de mélange d'air et de poudre (18) pour décharger le mélange d'air et de poudre du corps d'inhalateur (1);
une chambre de réception de poudre (5) adaptée pour recevoir une poudre, la chambre de réception de poudre (5) étant disposée dans le corps d'inhalateur (1) et communiquant avec l'extérieur du corps d'inhalateur (1) à travers le trajet d'entrée d'air (6);
un trajet de mélange d'air et de poudre (9) adapté pour transmettre le mélange d'air et de poudre circulant de la chambre de réception de poudre vers la sortie de mélange d'air et de poudre (18); et
une partie de dispersion (15) adaptée pour disperser la poudre dans le mélange d'air et de poudre passant par le trajet de mélange d'air et de poudre (9);
**caractérisé par**
un réservoir à mélange d'air et de poudre (12) disposé dans le trajet de mélange d'air et de poudre (9) entre la chambre de réception de poudre (5) et la partie de dispersion (15), le réservoir à mélange d'air et de poudre (12) étant adapté pour être en communication avec la chambre de réception de poudre (5) et l'extérieur du corps d'inhalateur (1), recevant ainsi une partie d'une dose de la poudre dans la chambre de réception de poudre (5) par une inhalation en une seule fois par l'utilisateur; et
un passage à air diluant (19) pour permettre la communication du réservoir à mélange d'air et de poudre (12) avec l'extérieur du corps d'inhalateur (1) et adapté pour introduire de l'air diluant dans le réservoir à mélange d'air et de poudre (12),
dans lequel la partie de dispersion (15) est adaptée pour recevoir du réservoir à mélange d'air et de poudre (12) la partie de la dose et pour fournir la partie dispersée de la dose à la sortie de mélange d'air et de poudre (18).

2. Inhalateur selon la revendication 1, comprenant, par ailleurs, un deuxième passage d'air diluant (20) adapté pour introduire de l'air diluant dans le trajet de mélange d'air et de poudre (9) en aval du réservoir à mélange d'air et de poudre (12) à l'écoulement du mélange d'air et de poudre du réservoir à mélange d'air et de poudre (12), et un régulateur (21) réglant de manière variable une surface d'ouverture du deuxième passage d'air diluant (20).

3. Inhalateur selon la revendication 1, dans lequel la partie de dispersion (15) comprend une pluralité de passages de dispersion ramifiés du trajet de mélange d'air et de poudre (9) en aval du réservoir à mélange d'air et de poudre (12), et une chambre de dispersion (17) disposée dans le trajet de mélange d'air et de poudre (9) en aval des passages de dispersion, chacun des passages de dispersion présentant une partie de passage de sortie qui est ouverte vers la chambre de dispersion (17) et disposée de manière à former un flux tourbillonnant du mélange d'air et de poudre.

4. Inhalateur selon la revendication 3, dans lequel la chambre de dispersion (17) a une section généralement circulaire et la partie de passage de sortie de chacun des passages de dispersion s'étend selon une direction tangentielle de la chambre de dispersion (17).

5. Inhalateur selon la revendication 1, dans lequel le trajet d'entrée d'air (6) est disposé de manière à permettre et bloquer la communication de fluide entre la chambre de réception de poudre (5) et l'extérieur du corps d'inhalateur (1).

6. Inhalateur selon la revendication 5, dans lequel le trajet d'entrée d'air (6) comprend au moins deux passages ayant une position d'alignement où les au moins deux passages sont alignés l'un par rapport à l'autre et une position décalée où les au moins deux passages sont désalignés l'un par rapport à l'autre.

7. Inhalateur selon la revendication 1, dans lequel le trajet de mélange d'air et de poudre (9) est disposé de manière à permettre et bloquer la communication de fluide entre la chambre de réception de poudre (5) et le réservoir à mélange d'air et de poudre (12).

8. Inhalateur selon la revendication 1, dans lequel le trajet de mélange d'air et de poudre (9) comprend au moins deux passages disposés entre la chambre de réception de poudre (5) et le réservoir à mélange d'air et de poudre (12), la pluralité de passages ayant une position d'alignement où les au moins deux passages sont alignés l'un par rapport à l'autre et une position décalée où les au moins deux passages sont décalés l'un par rapport à l'autre.

9. Inhalateur selon la revendication 3, comprenant, par ailleurs, un deuxième réservoir à mélange d'air et de poudre (14) adapté pour stocker temporairement le mélange d'air et de poudre s'écoulant du premier réservoir à mélange d'air et de poudre (12) vers les passages de dispersion de la partie de dispersion.

10. Inhalateur selon la revendication 1, dans lequel les passages de dispersion comprennent une entrée ouverte vers le deuxième réservoir à mélange d'air et de poudre (14).
